# EUROPEAN PATENT APPLICATION

(11) **EP 2 777 694 A1**
(43) Date of publication of application: **17.09.2014**
(21) Application number: 14158568.7
(22) Date of filing: 10.03.2014
(51) Int. Cl.: A61K 9/107, A61K 47/48, A61K 45/06

(54) **Hypoxia-Targeted Polymeric Micelles for Cancer Therapy and Imaging**

(30) Priority: 14.03.2013 US 201313804007
(71) Applicant: Giri, Brij P., Shelby Township, Michigan (US); Gregg, Kristina, Madison Heights, Michigan (US); Singh, Pritam, Shelby Township, Michigan (US); Dagli, Dinesh, Troy, Michigan (US); Gin, Anshu, Shelby Township, Michigan (US)
(72) Inventor: Giri, Brij P., Shelby Township, Michigan (US); Gregg, Kristina, Madison Heights, Michigan (US); Singh, Pritam, Shelby Township, Michigan (US); Dagli, Dinesh, Troy, Michigan (US); Gin, Anshu, Shelby Township, Michigan (US)
(74) Representative: Pfenning, Meinig & Partner GbR

(57) **Abstract**

The present invention provides a composition and method for targeting hypoxic tumor areas for detection or treatment or a treatment adjuvant for cancer. Specifically, a hypoxia targeting moiety is conjugated to a polymeric micelle containing imaging agents, therapeutic agents, or therapeutic adjuvants.

## Description

### CROSS-REFERENCE TO RELATED APPLICATION

This application is a completion application of co-pending United States Provisional Application Serial No. 61/620,620 filed April 5, 2012 for **"Hypoxia-Targeted Polymeric Micelles for Cancer Therapy and Imaging"** the entire disclosure of which is hereby incorporated by reference.

### BACKGROUND OF THE INVENTION

Cancer is a group of heterogeneous diseases that arise from increased cellular replication and decreased cell death. The key to finding successful treatments for cancer is to exploit differences that arise from normal and cancer cells to increase preferential cell killing. Many current chemotherapies and radiation therapy target rapidly growing cells. However, since there are normal cells that frequently divide, there are many side effects associated with the non-specificity of the treatments, such as myelosuppression, immunosuppression, and gastrointestinal complications. To minimize side effects, present strategies being investigated for cancer treatment are targeting deregulated proteins, gene therapy, or targeting differences in the microenvironment such as angiogenesis, pH, temperature, or hypoxia.

Hypoxia arises in tumors or metastases greater than 1 mm in diameter, due to inadequate vasculature. Hypoxia alters the tumor biology by increasing receptor tyrosine kinase activity, angiogenesis, invasiveness, metastasis, generation of reactive oxygen species, and suppression of immune reactivity. Hypoxia is generally regarded as a barrier to overcome, since it leads to resistance in many chemotherapies and radiation therapy. Currently, new approaches exploit hypoxia in tumors as a means of selective treatment, as disclosed by Denny (2000) Lancet Oncol, 1:25-29; Wilson & Hay (2011) Nat Rev Cancer, 11:393-410; Chen & Hu (2009) Med Res Rev, 29:29-64.\

There are a variety of compounds that selectively target and react within a hypoxic environment, including nitroaromatics, aromatic N-oxides, and aliphatic N-oxides. In US Patent No. 7,405,317 B2 and 7,550,496 B2 there is taught hypoxia targeting moieties for use as triggers in prodrug therapy.

Hypoxia targeting moieties have a high redox potential in which they may be reduced by any cellular environment. However, in the presence of oxygen the reaction is reversed. When hypoxia targeting moieties undergo reduction in hypoxic environments, they form covalent adducts with macromolecules such as DNA, proteins, or lipids, resulting in accumulation within the hypoxic environment. Reduction may be catalyzed by a specific enzyme or combination of enzymes such as cytochrome P-450, cytochrome P-450 reductase, xanthine oxidase, aldehyde oxidase, and DT-diaphorase see inter alia Workman (1992) Int J Radiat Oncol 22: 631-637.

As is known to those skilled in the art, nanocarriers are becoming increasingly popular in cancer treatments. Nanocarriers are nanoscale delivery vehicles (10-1000 nm diameter) in which molecules, such as drugs or imaging agents, may be encapsulated within or covalently linked to the exterior of the delivery vehicle. Examples include liposomes, micelles, dendrimers, and nanoemulsions. Nanocarriers can reduce many side effects of chemotherapies by limiting interactions with non-specific tissues. Nanocarriers are also useful in stabilizing, solubilizing, increasing circulation times, and improving biodistribution of drugs. They passively target tumors through the leaky vasculature, known as the enhanced permeability retention effect (EPR). They can also actively target tumors via surface modifications. EPR is based on the pathophysiological characteristics of tumors including hypervascularity, insufficient vessel maturation, secretion of vascular permeability factors, and inefficient lymphatic drainage, see inter alia Petros&DeSimone (2010) Nat Rev Drug Discov, 9:615-627.

Polymeric micelles (PMC) are supramolecular nanoparticles, which are useful agents in solubilizing and transporting hydrophobic drugs *in vivo.* PMCs comprise amphiphilic copolymers which are very stable in aqueous environments due to low critical micelle concentrations. A major advantage of PMCs is hydrophobic drugs can be encapsulated spontaneously, eliminating the need to alter the drug with potentially detrimental modifications. Effective PMCs can be optimized for biocompatibility, stability, drug loading capacity and release kinetics, size (10-100nm), and an appropriate clearance mechanism. These characteristics are optimized through selection of core forming units (hydrophobic) and the corona forming units (hydrophilic), the arrangement of the monomers into the polymer, and the methods of polymerization and micellization. PMCs can also be produced in large quantities with reproducible results and are tunable for size and composition. As drug delivery systems, PMCs can lower drug toxicity by limiting interactions with nonspecific cells, increase drug circulation time, increase drug stability and solubility, and can be modified with targeting moieties, as reported by Torchilin (2001) J Control Release, 73:137-172. Several chemotherapeutic agents have been successfully encapsulated with high efficiency in PMCs, some of which are currently in clinical trials. See, inter alia, Rios-Doria et al. 2012, Drug Deliv, 2012:1-8; Matsumura 2008, Jpn J Clin Oncol, 38:793-802; US Patent No. 6,322,817 B1; US Patent Application Publication No. 2010/0158850 A1; and Clinical Trials ID NCT00912639; NCT00886717; NCT01426126.

Photodynamic therapy (PDT) has been shown to be a very effective treatment of certain cancers with limited side effects (Dolmans et al. (2003) Nat Rev Cancer, 3:380-387). The principle of PDT is to initially sensitize cells with a photosensitizer, and then introduce light to the targeted area in the presence of oxygen to generate reactive oxygen species (ROS), thereby inducing cell death. PDT has a low toxicity compared to other cancer therapies since the photosensitizer is non-toxic under dark conditions and may be triggered by specific light wavelengths, and both the sensitizer and light may be preferentially directed to the target area sparing non-specific tissues. Light sources for PDT typically are in the higher wavelength range for enhanced tissue penetration and may be administered by optic fiber to maximize the amount of tissue that can be treated with light. However, PDT is still only efficacious for superficial tumors. By creating a light supply that can illuminate deeply embedded tumors and penetrate throughout the surrounding areas, the applications of PDT can be broadened significantly. Light can be generated throughout the tumor by utilizing a chemiluminescent producing system (CLS), or the generation of light by chemical reactions. Laptev et al. (2006) Br J Cancer, 95:189-196 demonstrated efficacy of PDT in leukemic cells by utilizing intracellular chemiluminescence through a luminol reaction.

In US Patent No. 7,416,898 B2, the disclosure of which hereby incorporated by reference, there is disclosed highly sensitive chemiluminescent substrates that can be modified to be triggered by different mechanisms, such as by enzymatic reactions, temperature and pH differentials, and others. The 1,2-dioxetane chemiluminescent chemical reaction may be enhanced by methods in US Pat no. 7,300,766 B2 the disclosure of which is hereby incorporated by reference, or by utilization of organic molecules such as diphenylanthracene and fluorescein.

Enhancing the chemiluminescent reaction facilitates more effective PDT by increasing the ability to activate the photosensitizers. For example, fluorescein can enhance the 1,2-dioxetane chemiluminescence and then transfer energy to a photosensitizer, such as Rose Bengal. Another benefit of PDT as a therapy is the ability to track the location of the effective treatment area due to fluorescence emission of the photosensitizers.

### SUMMARY OF THE INVENTION

The present invention as detailed below, improves upon the prior art by providing compositions and methods for treatment and detection of hypoxic areas of cancers with a targeted polymeric micelle (PMC).

The present invention provides a hypoxia targeting moiety attached to a PMC, which is capable of encapsulating a chemotherapeutic agent, a light producing system, a sensitizing system, a radioactive system, and the like.

Accordingly, the present invention provides a compound of the following formula: 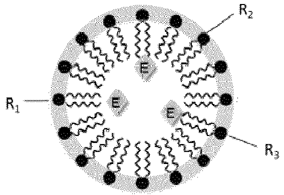
where E is an encapsulated agent within a PMC in which E is selected from the group consisting of an imaging agent, a therapeutic agent, a therapeutic adjuvant, a light producing system, a radioactive system, a sensitizing agent, and the like. The encapsulated agent may or may not be conjugated to a hypoxia targeting moiety;
R₁ is a hypoxia targeting moiety including an aromatic N-oxide, an aliphatic N-oxide, nitroazole, nitroimidazole, nitrothiophene, nitrothiazole, nitrooxazole, nitrofuran, nitropyrrole, transition metal moieties and the like;
R₂ maybe an imaging agent, a targeting moiety, a therapeutic agent, a sensitizing agent, or any combination thereof. R₂ may be the same as the encapsulated compound or a different compound, and
R₃ is a polar biocompatible moiety for improving solubility, stability, and biodistribution of the PMC.

The present invention also provides a method for treating tumors and for generating light using the above compound.

For a more complete understanding of the present invention reference is made to the following detailed description and accompanying examples.

### DESCRIPTION OF THE PREFERRED EMBODIMENT

In the ensuing description, the following definitions will be used for the purpose of clarity.

"Tumor" or "cancer" refers to a heterogeneous group of proliferative diseases resulting in a solid mass of cells. This includes metastases that may arise from cells that have migrated from the primary mass to form secondary or multiple masses.

"Therapeutic" or "therapeutic agent" or "drug" refers to any agent used in the treatment of cancer.

"Sensitizer" or "sensitizing agent" refers to any agent that makes cells more susceptible to a treatment. Included within this class of agents are photosensitizers whereby cells undergo programmed cell death in the presence of light.

"Hypoxia" or "tumor hypoxia" refers to lowered levels of oxygen in cells and tissues due to insufficient blood supply within the tumor microenvironment.

"Polymeric micelle" or "PMC" refers to supramolecular structures comprising amphiphilic co-polymers which, when exposed to an aqueous environment, produce a hydrophobic interior core and a stabilizing hydrophilic corona.

"Imaging agent" is any compound that may be used for aiding in tumor detection and diagnosis, and which may be used in a variety of applications including, but not limited to MRI scans, PET scans, CAT/CT scans, fluorescence tomography, fluorescent reflectance, radiology, NMR spectroscopy, microscopy, histology and the like.

"Encapsulated" refers to the position of a molecule within the core or within the core-corona interface of the PMC, and which is protected from the surrounding environment. An encapsulated agent may be held within the PMC through hydrophobic interactions, hydrogen bonding, ionic bonding, or covalent bonding.

As noted hereinabove, the present invention provides a compound of the following formula: 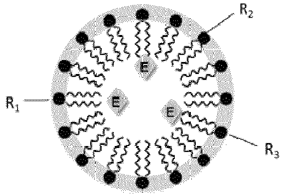
where R₁ is a hypoxia targeting moiety;
R₂ is an imaging agent, a targeting moiety, a therapeutic agent, a sensitizing agent or mixtures. R₂ may be the same as or different from the encapsulated compound;
R₃ is a polar biocompatible moiety to improve solubility, stability, and biodistribution of a PMC, and
E is an encapsulated agent within a PMC in which E is selected from the group consisting of an imaging agent, a therapeutic agent, a therapeutic adjuvant, a light producing system, a radioactive system, a sensitizing agent, and the like. The encapsulated agent may or may not be conjugated to the hypoxia targeting moiety.

### Polymeric Micelle

The PMCs of the present invention are generally from about 10 to about 100nm in diameter and comprise hydrophobic biocompatible polymers forming the core and hydrophilic or polar biocompatible polymers forming the corona.

Biocompatible polymers that may be used to form the core of the PMC include, for example, polystyrene, poly(divinylbenzene), poly(acrylate), polymethylmethacrylate, poly(hydroxyethyl methacrylate), poly(vinyltoluene), poly(butadiene), poly(aspartic acid), poly(benzyl aspartate), polycaprolactone and derivatives thereof, poly(lactide) and derivatives thereof, poly(benzyl glutamate), poly(L-lysine), poly(propylene oxide), oligo(methyl methacrylate), poly(isoprene), poly(isopropyl acrylamide), calixarenes, polyanhydrides, pseudo-poly(amino acids), polyphosphazenes and derivatives thereof and the like, as well as mixtures thereof.

Hydrophilic or polar biocompatible polymers that may be used to form the corona of the PMC include, for example, poly(ethylene glycol), poly(vinyl alcohol), poly(acrylic acid), poly(methacrylic acid), poly(acrylamide), poly(vinyl pyrrolidone), poly(ethylene oxide), poly(propylene oxide), poly(vinylmethyl ether), hydroxypropyl cellulose, chitosans, polysaccharides, tertiary ammonium and phosphonium salts, and the like, as well as mixtures thereof.

Among the useful core polymers are those derived from a corepoly (vinyl benzyl) polymer coreand which correspond to the formula: 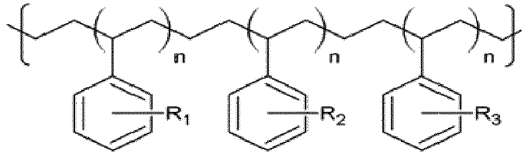 where n is an integer from 1 to 20; R₁ represents the hypoxia targeting moiety; R₂ is the imaging or contrast agent, the targeting moiety, the therapeutic agent, the sensitizing agent, or the like as defined above; and R₃ is the polar biocompatible moiety.

The hypoxia targeting moieties maybe an aromatic N-oxide, an aliphatic N-oxide, nitroazole, nitroimidazole, nitrothiophene, nitrothiazole, nitrooxazole, nitrofuran, nitropyrrole, transition metal moieties, and the like.

Useful imaging or contrast agents may be selected from the group consisting of fluorophores, dyes, quantum dots, transition metal, transition metal complexes, radionuclides, and the like, as well as mixtures thereof.

A secondary targeting moiety, in addition to the hypoxia targeting moiety, maybe used herein. Such secondary targeting moiety may be selected from group consisting of an antibody or modified antibodies such as antigen binding fragments (Fab) or single chain variable fragments (scFv) to a molecular marker, a ligand for a cell surface receptor, a peptide, a protein, a glycoprotein, a nucleic acid sequence, a carbohydrate, a steroid, and the like as well as mixtures thereof.

Useful therapeutic agents may be selected from the group consisting of chemotherapeutics, radioisotopes, therapeutic proteins or peptides, gene therapy, and the like.

Useful chemotherapeutics are selected from the group consisting of antimetabolites, alkylating agents, alkaloids, topoisomerase inhibitors, kinase inhibitors, angiogenesis inhibitors, cytotoxic antibiotics, platinum based drugs and the like.

Sensitizing agents may be selected from the group containing photosensitizers such as napthalenes, anthracenes, biphenyls, quinones, porphyrins, and phthalocyanins, fluorescein, Rose Bengal, eosin blue, and erythrosin B, oxygen carriers such as endoperoxides and nitroxides, as well as mixtures thereof.

Polar biocompatible moieties may be selected from the group containing such as poly(ethylene glycol), poly(vinyl alcohol), poly(acrylic acid), poly(methacrylic acid), poly(acrylamide), poly(vinyl pyrrolidone), poly(ethylene oxide), poly(propylene oxide), poly(vinylmethyl ether), hydroxypropyl cellulose, chitosans, polysaccharides, tertiary ammonium and phosphonium salts, and the like, as well as mixtures thereof.

A preferred polar biocompatible moiety is a tertiary salt represented as: 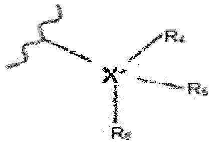 where X is either N or P; R₄, R₅, and R₆ each represents a straight or branched alkyl chain of 1-20 carbon atoms, unsubstituted or substituted with one or more hydroxyl, alkoxy, aryloxy, amino or substituted amino groups, fluoroalkane, p-fluoroaryl, deuterated alkyl groups, and the like and mixtures thereof.

Another useful copolymer is cross-linked with a linker region which corresponds to the formula: 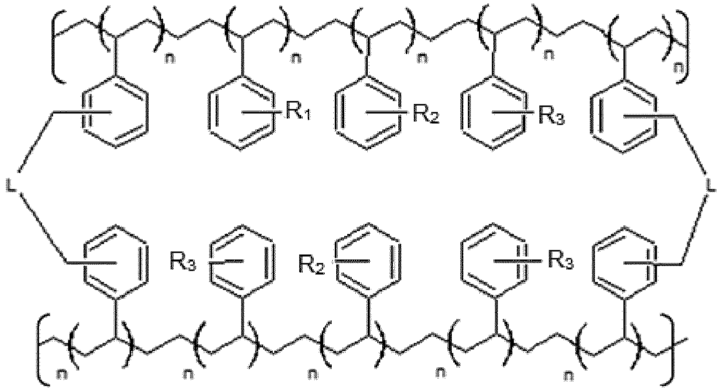 where n is an integer from 1 to 20; R₁, R₂, and R₃ are as defined above; L is a linker region of saturated or unsaturated carbons attached to trisubstituted amines or trisubstitutedphosphines, as disclosed in US Patent No. 7,300,766 B2.

The linker region is preferably selected from the group consisting of N,N,N'N'-tetramethyl-2-butene-1,4-diamine; N,N,N'N'-tetramethylbutane-1,4-diamine; 1,4-dimethyl piperazine; 1,4-phenylenediamine and mixtures thereof.

### Hypoxia Targeting Moieties

As noted above, the present invention utilizes hypoxia targeting moieties linked to PMCs for targeted delivery to tumors. The hypoxia targeting moieties are structures which preferentially accumulate within a hypoxic environment over areas with sufficient oxygen supply.

The hypoxia targeting moieties are represented by the following formula:

R - N → 0

where R is a cyclic or linear aliphatic moiety or aromatic moiety.

As noted above, among the useful hypoxia targeting moieties include an aromatic N-oxide, an aliphatic N-oxide, nitroazole, nitroimidazole, nitrothiophene, nitrothiazole, nitrooxazole, nitrofuran, nitropyrrole, transition metal moieties, and the like.

A preferred hypoxia targeting moiety may be represented by the following structure: 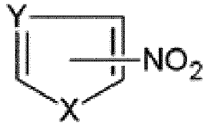 where X is N, S, or O and Y is C or N.

In another preferred embodiment the moiety is a substituted or unsubstituted 2-nitroimidazole when both X and Y are N, and which corresponds to the following: 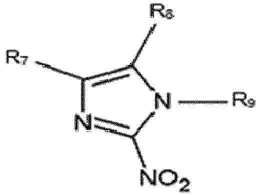 where R₇, R₈, and R₉, individually, represent attachment to a PMC directly or through a linker region; a deuterated or non-deuterated alkyl, a carboxylate, an alkyl carboxylate, an amino, a substituted or unsubstituted aryl, a substituted or unsubstitutedheteroaryl, and the like, as well as mixtures thereof.

### Encapsulated and Functional Moieties

As noted above, PMCs may have functional groups covalently attached or hydrophobic agents encapsulated within the PMC, including therapeutic agents, singlet oxygen producing systems, light producing systems, radioactive systems, sensitizing agents, imaging agents, and the like, as well as mixtures thereof.

Useful hydrophobic therapeutic agents that may be encapsulated within the PMC may include, for example, chemotherapeutic drugs, angiogenesis inhibitors, radiotoxins, and others. Chemotherapeutic drugs include for example, doxorubicin, daunorubicin, epirubicin, cisplatinum, carboplatin, paclitaxel, camptothecin, and others. Preferably the encapsulated agent is highly hydrophobic and otherwise insoluble in an aqueous medium.

A preferred PMC in accordance herewith is a hypoxia targeted therapeutic nanocarrier with 2-nitroimidazole as the hypoxia targeting moiety, a trisubstituted salt as the polar biocompatible component, and one or more encapsulated therapeutic agents, represented to maybe as follows:

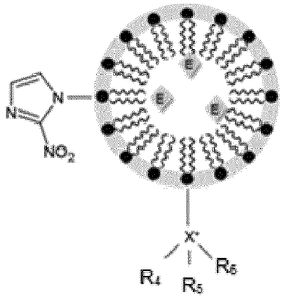

In another preferred embodiment the PMC nanocarrier is modified with an imaging agent for in vivo imaging, and corresponds to the following structure: 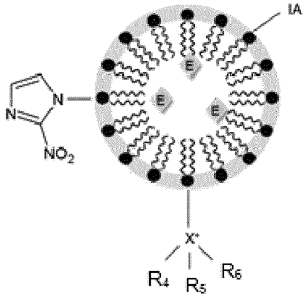 where IA represents an imaging agent including fluorescent moieties, deuterated moieties, electromagnetic moieties, radioisotopes, and others.

Suitable fluorescent moieties include, for example, organic dyes, quantum dots, fluorescent probes, and fluorescent biomolecules, such as, proteins and peptides. The imaging agents are covalently linked to the PMC and may be located outside the PMC (hydrophilic) or within the PMC (hydrophobic), or non-covalently linked and encapsulated within the PMC (hydrophobic).

A secondary targeting moiety, in addition to the hypoxia targeting moiety, may be used herein. Such secondary targeting moiety may be selected from group consisting of an antibody or modified antibodies such as antigen binding fragments (Fab) or single chain variable fragments (scFv) to a molecular marker, a ligand for a cell surface receptor, a peptide, a protein, a glycoprotein, a nucleic acid sequence, a carbohydrate, a steroid, and the like as well as mixtures thereof.

### Photodynamic Therapy

The success of PDT depends on sufficient accumulation of a photosensitizer in the target area and which is activated by an appropriate light source.

As noted above, the present invention may be used for PDT to deliver a sensitizer, a chemiluminescent substrate as the light source, or both. The present invention also provides a method for treating tumors and for generating light using chemiluminescence.

Photosensitizers may be organic dyes and derivatives thereof, napthalenes, anthracenes, biphenyls, quinones, porphyrins, and phthalocyanins, as well as mixtures thereof. Organic dyes include fluorescein, Rose Bengal, eosin blue, erythrosin B, and the like.

The present invention also provides a method for the generation of light selectively within the tumor microenvironment in the presence of the photosensitizers. The aforementioned hypoxia targeted PMC may be used to encapsulate a chemiluminescent substrate and allow sufficient accumulation within the tumor microenvironment where the substrate may be triggered, thus activating the photosensitizer already present in the tumor.

Suitable chemiluminescent substrates that may be encapsulated include stabilized 1,2-dioxetanes such as described in US patent no. 7,416,898 B2, the disclosure of which is hereby incorporated by reference. Another suitable chemiluminescent substrate is luminol.

A preferred embodiment of the present invention when used in PDT can be shown as: 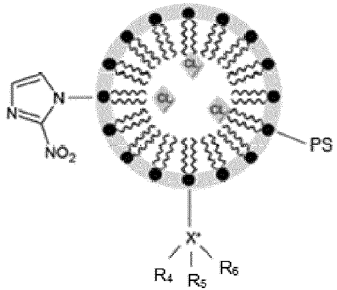 wherein the PMC is conjugated to 2-nitroimidazole, PS is a photosensitizer selected from the group consisting of napthalenes, anthracenes, biphenyls, quinones, porphyrins, phthalocyanins, fluorescein, fluorescein derivatives, Rose Bengal, eosin blue, and erythrosin B, X⁺ is a tertiary salt of either N or P, and CL is an encapsulated chemiluminescent substrate. Some of the useful CL substrates are 1, 2-dioxetane compounds and luminol. The PMC is directed to the tumor and retained within via the 2-nitroimidazole, where the CL is triggered. The chemiluminescent light excites the photosensitizer to induce apoptosis or necrosis in nearby cells.

A preferred embodiment of the present invention for use in PDT and tumor imaging can be shown as: 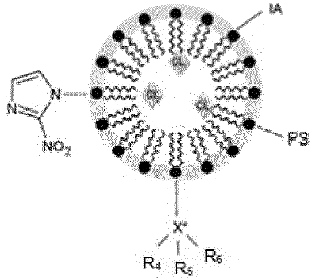 where IA, PS, and X⁺ are as described above.

For a more complete understanding of the present invention, reference is made to the following non-limiting examples. In the examples all parts are by weight absent contrary indications.

### Example I

This example illustrates the preparation of a PMC targeted to hypoxia.

### Step 1: Synthesis of 1-(3-phthalimidopropyl)-2-nitroimidazole

A mixture of 5.36 parts 2-nitroimidazole, 13.39 parts N-(3-bromopropyl)-phthalimide and 11.85 parts N,N-diisopropylethylamine is heated at reflux in a preheated oil bath maintained between 155-160°C. under an argon atmosphere for 3 hours and 45 minutes. The mixture is allowed to cool to room temperature and solidify. The solid is broken up and stirred with DI water. The solid is air-dried for 4 hours at room temperature followed by at 40°C. for 18 hours. The structure is confirmed by NMR spectroscopy.

### Step 2: Synthesis of 1-(3-Aminopropyl)-2-nitroimidazole

A solution of 0.15 parts anhydrous hydrazine in anhydrous ethanol (1 mL) is added to a refluxing solution of 0.14 parts 1-(3-phthalimidopropyl)-2-nitroimidazole obtained in step 1 in anhydrous ethanol (5mL) and refluxed for 2 hours. The mixture is concentrated to dryness at ∼30 °C. under reduced pressure. The residue is slurried with dichloromethane (20mL) for 30 minutes, and the solid is filtered and washed twice with dichloromethane (5 mL). The filtrate is combined and concentrated to dryness at ∼30 °C and dried under vacuum for 1 hour.

A solution of 10 parts 1-(3-aminopropyl)-2-nitroimidazole in dry DMF (2mL) is added to a solution of poly(vinylbenzylchloride) in DMF (100mL) under argon atmosphere and stirred for 18 hours. The solution is heated to 50-55°C. for 3 hours. The mixture is cooled to room temperature for 3 hours. Tributylphosphene (23mL) is added to the solution and stirred for 18 hours. The solution is heated to 55-57°C. for 3 hours and cooled to room temperature for 3 hours. Trioctylphosphene (5.5mL) is added to the solution and stirred for 66 hours.

The solution is heated to 50-55°C. for 4 hours and cooled to room temperature for 18 hours. The solution is concentrated to 50 mL under vacuum while heated to 30-35°C and cooled to room temperature. Dry DMF (30mL) is added and the solution poured slowly to anhydrous ether (1.5L) and is vigorously stirred for 30 minutes. The solid is washed with anhydrous ether (3 x 500mL) and vigorously stirred for 10 minutes and decanted each time. The solid is gravity filtered and dried under vacuum for 24 hours at 25°C.

### Example II

This example illustrates the preparation of a PMC targeted to hypoxia modified with the covalent attachment of an imaging agent and photosensitizer.

### Synthesis of 1-(3-Aminopropyl)-2-nitroimidazole with Rose Bengal and Fluorescein

A solution of 0.15 parts anhydrous hydrazine in anhydrous ethanol (1 mL) is added to a refluxing solution of 0.14 parts 1-(3-phthalimidopropyl)-2-nitroimidazole obtained in step 1 of Example I in anhydrous ethanol (5mL) and refluxed for 2 hours. The mixture is concentrated to dryness at ∼30 °C. under reduced pressure. The residue is slurried with dichloromethane (20mL) for 30 minutes, and the solid filtered and washed twice with dichloromethane (5 mL). The filtrate is combined and concentrated to dryness at ∼30 °C and dried under vacuum for 1 hour.

A solution of 10 parts 1-(3-aminopropyl)-2-nitroimidazole in dry DMF (2mL) is added to a solution of poly(vinylbenzylchloride) in DMF (100mL) under argon atmosphere and stirred for 18 hours. The solution is heated to 50-55°C. for 3 hours. The mixture is cooled to room temperature for 3 hours and 0.13 parts Rose Bengal is added. The mixture is stirred for 18 hours. The solution is heated to 50-55°C. for 3 hours and cooled to room temperature for 3 hours. To this solution 0.07 parts fluorescein is added and stirred for 18 hours. The solution is heated to 50-55°C. for 3 hours and cooled to room temperature for 3 hours. Tributylphosphene (23mL) is added to the solution and stirred for 18 hours. The solution is heated to 55-57°C. for 3 hours and cooled to room temperature for 3 hours. Trioctylphosphene (5.5mL) is added to the solution and stirred for 66 hours.

The solution is heated to 50-55 °C. for 4 hours and cooled to room temperature for 18 hours. The solution is concentrated to 50 mL under vacuum while heating to 30-35°C and cooled to room temperature. Dry DMF (30mL) is added and the solution poured slowly to anhydrous ether (1.5L). The solution is vigorously stirred for 30 minutes. The solvent is decanted and the solid washed with anhydrous ether (3 x 500mL) with vigorous stirring for 10 minutes. The solid is gravity filtered and dried under vacuum for 24 hours at 25 °C (22.5 parts of product recovered).

### Example III

This example illustrates encapsulation of a hydrophobic drug within a PMC.

The PMC from Example I is used to encapsulate the following drug:

### Drug A: (3Z)-3-{[3,5-dimethyl[propyl-1-ylcarbamoyl]-1H-pyrrol-2 methylidene}-5-fluoro-1,3-dihydro-2H-indol-2-one

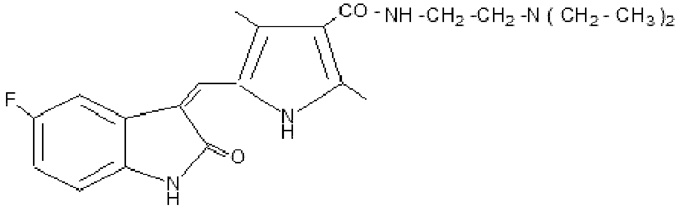

The polymer is dissolved indeionized water or an aqueous buffer at a concentration of 0.010 g/mL. Drugs A is dissolved in DMSO at a concentration of 5 µg/µl. Drug A is added dropwise to the polymer solution with constant stirring at room temperature. The drugs are added over the course of 60 minutes resulting in a 2-5 fold molar excess over polymer. The solution is centrifuged to remove any precipitates and transferred to dialysis tubing with 10,000 MWCO. The solution is dialyzed against an aqueous buffer(50x volume) for 30 minutes.

The absorbance of the polymer-drug solution and dialysis buffer are recorded and compared to respective pre-dialysis samples by a UV/Vis spectrophotometer (polymer absorbance = 280nm; Drug A absorbance = 450nm). The encapsulation efficiency is calculated from the change in absorbance of the drug after this initial dialysis. The polymer-drug solution is dialyzed further with fresh buffer (50x volume) with multiple readings over the course of 72 hours with 4 buffer changes. The rate at which the drug is released from the polymeric micelle is calculated by the change in absorbance of the drug over time from the polymer-drug solution and dialysis buffer.

Encapsulation efficiency for Drug A is calculated to be greater than 80% in a tris buffer (pH 7.4) with 50% drug released over a 24 hour time course.

### Example IV

This example illustrates encapsulation of a hydrophobic drug within a PMC.

The PMC from Example I is used to encapsulate the following drug:

### Drug B: N-(4-chloro-3-trifluoromethyl) phenyl-N'-{4-methylcarbamoyl]-4-pyridyloxyphenyl} urea:

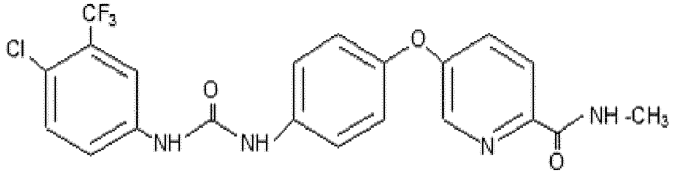

Encapsulation of Drug B is carried out by the same dialysis procedure as described in Example III for Drug A. Drug B is dissolved in DMSO to a stock solution of 2.5mg/ml. The absorbance of Drug B is followed at 310nm and the polymer at 280nm. For Drug B encapsulation efficiency is calculated to be greater than 85% with 20% of the drug released after 24 hours.

### Example V

This example illustrates the preparation of an encapsulated chemiluminescent substrate in a singlet oxygen producing PMC

The polymer described in Example I is used to encapsulate a 1,2-dioxetane chemiluminescent substrate for phosphatases.

A 1,2-dioxetane is added to an aqueous solution with the polymer from Example 1 and dialyzed in a membrane with a 10,000 MWCO against an AP stabilizing tris buffer (50x volume) for 24 hours at room temperature. The encapsulation efficiency is determined by change alkaline phosphatase activity after dialysis.

### Example VI

This example illustrates the preparation of an encapsulated chemiluminescent substrate in a singlet oxygen producing PMC

The polymers described in Example II is used to encapsulate a 1,2-dioxetane chemiluminescent substrate for phosphatases.

A 1,2-dioxetane is added to an aqueous solution with the polymer from Example II and dialyzed in a membrane with a 10,000 MWCO against an AP stabilizing tris buffer (50x volume) for 24 hours at room temperature. The encapsulation efficiency and stability of the complex is determined by monitoring the change in alkaline phosphatase activity and the absorbance readings over time (polymer = 290 nm, fluorescein = 495 nm, Rose Bengal = 570 nm).

### Example VII

This example illustrates encapsulation of hydrophobic fluorophores within a PMC.

The polymer from Example I is used to encapsulate fluorescein and Rose Bengal. The polymer is dissolved in an aqueous buffer near physiologic conditions at a concentration of 0.010 g/mL. Stock solutions of fluorescein and Rose Bengal are prepared in DMSO. Each fluorophore is added dropwise to the polymer solution with constant stirring at room temperature until final concentration of fluorescein is 0.167µM and Rose Bengal is 13.7µM. The solution is allowed to mix for 2 hours at room temperature. Figure 1 shows the change in fluorescence intensity and red shifted spectra when the fluorophores are encapsulated in the polymer compared to the fluorophores in aqueous solution. The energy transfer from fluorescein to Rose Bengal is enhanced more than 1000-fold when encapsulated in the polymer. The encapsulated fluorophores are dialyzed in 100x volume aqueous buffer for 24 hours in dialysis tubing with MWCO of 10,000. Greater than 90% of the fluorophores are retained in the polymer within the dialysis tubing as determined by fluorescence intensities and absorbance.

### Example VIII

This example illustrates the encapsulation of hypoxia-targeting hydrophobic drugs encapsulated within a hypoxia-targeting PMC.

The PMC from Example I is used to ecapsulate the following hypoxia-targeting drug:

### Drug C: (3Z)-3-{[3,5-dimethyl[3-(2-nitroimidazole)-propyl-1-ylcarbamoyl]-1 H-pyrrol-2-ylmethylidene}-5-fluoro-1,3-dihydro-2H-indol-2-one

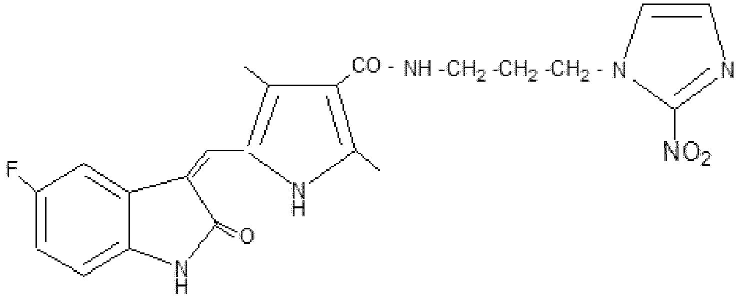

The drug is dissolved in DMSO at a stock concentration 10x the intended final encapsulated concentration. The drug is added dropwise to an aqueous solution of the polymer from Example I, and allowed to mix for 2 hours. Excess drug is removed by dialysis, and encapsulation efficiency is calculated from the change in absorbance.

### Example IX

This example illustrates the encapsulation of hypoxia-targeting hydrophobic drugs encapsulated within a hypoxia-targeting PMC.

Following the procedure of Example VIII, the polymer of Example I is used to encapsulate the following drug:

Drug D: N-(4-chloro-3-trifluoromethyl) phenyl-N'-{4-[3-(2-nitroimidazole) carbamoyl]-4-pyridyloxyphenyl} urea

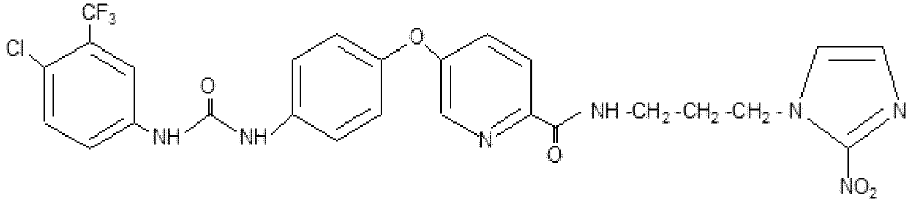

The present invention improves therapeutic efficacy of different cancer treatments by providing a targeted delivery system for therapeutics. Improved efficacy is achieved by increasing solubility and stability of the drug, improving bioavailability, and reducing toxicity. The present invention may also be used for imaging and locating the disease to aid in treatment decisions.

Having, thus, described the invention, what is claimed is:
The present invention further comprises the following aspects:
1. A polymeric micelle corresponding to the formula: 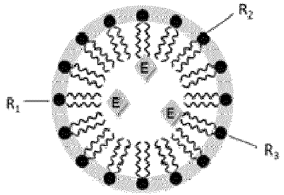
   where E is an encapsulated agent within a polymeric micelle (PMC) in which E is selected from the group consisting of an imaging agent, a therapeutic agent, a therapeutic adjuvant, a light producing system, a radioactive system, a sensitizing agent, and mixtures thereof;
   R₁ is a hypoxia targeting moiety including aromatic N-oxide, aliphatic N-oxide, nitroazole, nitroimidazole, nitrothiophene, nitrothiazole, nitrooxazole, nitrofuran, nitropyrrole, and transition metal moieties;
   R₂ is selected from the group consisting of an imaging agent, a targeting moiety, a therapeutic agent, a sensitizing agent, and mixtures thereof;
   R₃ is a polar biocompatible moiety for improving solubility, stability, and biodistribution of the micelle, and
   wherein the sensitizing agent may be conjugated to the hypoxia targeting moiety.
2. The micelle of aspect 1 wherein:
   the polymeric micelle has a size ranging from about 10 nm to about 100 nm in diameter and comprises a hydrophobic biocompatible core and a hydrophilic or polar biocompatible corona.
3. The micelle of aspect 2 wherein:
   the biocompatible polymer forming the core of the PMC is selected from the group consisting of polystyrene, poly(divinylbenzene), poly(acrylate), polymethylmethacrylate, poly(hydroxyethyl methacrylate), poly(vinyltoluene), poly(butadiene), poly(aspartic acid), poly(benzyl aspartate), polycaprolactone and derivatives thereof, poly(lactide) and derivatives thereof, poly(benzyl glutamate), poly(L-lysine), poly(propylene oxide), oligo(methyl methacrylate), poly(isoprene), poly(isopropyl acrylamide), calixarenes, polyanhydrides, pseudo-poly(amino acids), polyphosphazenes and derivatives thereof and mixtures thereof.
4. The micelle of aspect 1 wherein:
   The corona forming biocompatible polymeris selected from the group consisting of, poly(ethylene glycol), poly(vinyl alcohol), poly(acrylic acid), poly(methacrylic acid), poly(acrylamide), poly(vinyl pyrrolidone), poly(ethylene oxide), poly(propylene oxide), poly(vinylmethyl ether), hydroxypropyl cellulose, chitosans, polysaccharides, tertiary ammonium and phosphonium salts, and mixtures thereof.
5. The micelle of aspect 1 which corresponds to the formula: 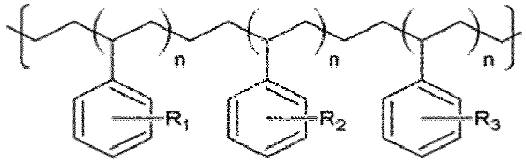 where n is an integer from 1 to 20.
6. The micelle of aspect 5 wherein:
   the hypoxia targeting moiety is selected from the group consisting of aromatic N-oxide, aliphatic N-oxide, nitroazole, nitroimidazole, nitrothiophene, nitrothiazole, nitrooxazole, nitrofuran, nitropyrrole, transition metal moieties, and mixtures thereof.
7. The micelle of aspect 5 wherein:
   the imaging or contrast agent is selected from the group consisting offluorophores, dyes, quantum dots, transition metal, transition metal complexes, radionuclides, and mixtures thereof.
8. The micelle of aspect 5 wherein:
   the therapeutic agent is selected from the group consisting of chemotherapeutics, radioisotopes, therapeutic proteins or peptides, gene therapy, and mixtures thereof.
9. The micelle of aspect 8 wherein:
   the chemotherapeutic compound is selected from the group consisting of antimetabolites, alkylating agents, alkaloids, topoisomerase inhibitors, kinase inhibitors, angiogenesis inhibitors, cytotoxic antibiotics, platinum based drugs and mixtures thereof.
10. The micelle of aspect 5 wherein:
   the chemotherapeutic compound is selected from the group consisting of antimetabolites, alkylating agents, alkaloids, topoisomerase inhibitors, kinase inhibitors, angiogenesis inhibitors, cytotoxic antibiotics, platinum based drugs and mixtures thereof.
11.The micelle of aspect 5 wherein:
   the sensitizing agent is selected from the group consisting of (a) photosensitizers, including naphthalene, anthracene, biphenyl, quinone, porphyrin, and phthalocyanins, (b) fluorescein, (c) Rose Bengal, (d) eosin blue, (e) erythrosin B, (f) oxygen carriers including endoperoxides and nitroxides, and (g) mixtures thereof.
12. The micelle of aspect 5 wherein:
   the polar biocompatible moietyisselected from the group consisting of poly(ethylene glycol), poly(vinyl alcohol), poly(acrylic acid), poly(methacrylic acid), poly(acrylamide), poly(vinyl pyrrolidone), poly(ethylene oxide), poly(propylene oxide), poly(vinylmethyl ether), hydroxypropyl cellulose, chitosans, polysaccharides,tertiary ammonium and phosphonium salts, and mixtures thereof.
13. The micelle of aspect 12 wherein the polar biocompatible moiety is a tertiary salt corresponding to the formula: 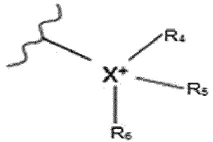 where X is either N or P; R₄, R₅, and R₆ are each,individually,a straight or branched alkyl chain of 1-20 carbon atoms, unsubstituted or substituted with one or more hydroxyl, alkoxy, aryloxy, amino or substituted amino groups, fluoroalkane, p-fluoroaryl, deuterated alkyl groups, and mixtures thereof.
14. The micelle of aspect 1 which corresponds to the formula: 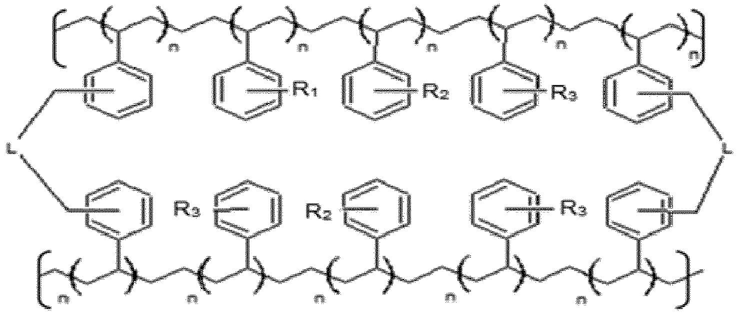 wherein L is a linker region of saturated or unsaturated carbons attached to trisubsituted amines or trisubstituted phosphines, and n is an integer from 1 to 20.
15. The micelle of aspect 1 wherein R₁ corresponds to the formula: 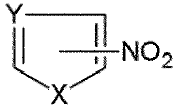 where X is N, S, or O and Y is C or N.
16. The micelle of aspect 15 wherein the hypoxia targeting moiety is a substituted or unsubstituted 2-nitroimidazole and when both X and Y are Ncorresponds to the following: 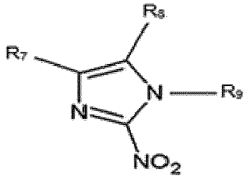 where R₇, R₈, and R₉, individually, represent attachment to a PMC directly or through a linker region; a deuterated or non-deuterated alkyl, a carboxylate, an alkyl carboxylate, an amino, a substituted or unsubstituted aryl, a substituted or unsubstitutedheteroaryl, and mixtures thereof.
17. The micelle of aspect 12 which corresponds to formula: 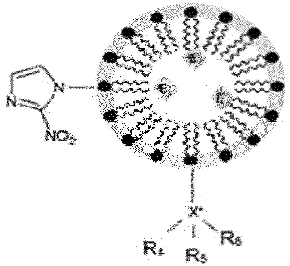 wherein 2-nitroimidazole is the targeting moiety, the polar biocompatible component is a tertiary salt, E is a therapeutic agent,X is either N or P, R₄, R₅, and R₆ are, individually, a straight or branched alkyl chain of 1-20 carbon atoms, unsubstituted or substituted with one or more hydroxyl, alkoxy, aryloxy, amino or substituted amino groups, fluoroalkane, p-fluoroaryl, deuterated alkyl groups, and mixtures thereof.
18. The micelle of aspect 17 which further comprises an imaging agent, the moiety corresponding to the formula: 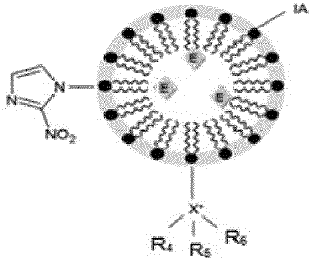 where IA represents an imaging agent including fluorescent moieties, deuterated moieties, electromagnetic moieties, radioisotopes, and mixtures thereof.
19. The micelle of aspect 17 wherein the imaging agent is a fluorescent moietyselected from the group consisting of:
   organic dyes, quantum dots, fluorescent probes, and fluorescent biomolecules, the imaging agents being covalently linked to the PMC either outside the PMC (hydrophilic), within the PMC (hydrophobic), or non-covalently linked to the PMC or encapsulated within the PMC (hydrophobic).
20. A photodynamic therapy polymeric micelle corresponding to the formula: 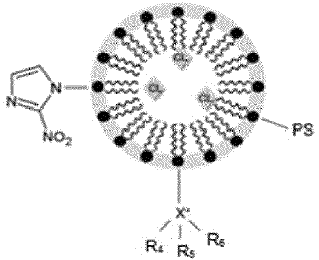 wherein the PMC is conjugated to 2-nitroimidazole, PS is a photosensitizer selected from the group consisting of napthalenes, anthracenes, biphenyls, quinones, porphyrins, phthalocyanins, fluorescein, fluorescein derivatives, Rose Bengal, eosin blue, and erythrosin B, X+ is a tertiary salt of either N or P, and CL is an encapsulated chemiluminescent substrate, the substrate being selected from the group consisting of substrates, 1,2-dioxetane compounds and luminol, 2-nitroimidazole, where the CL is triggered, R₄, R₅, and R₆ are, individually, a straight or branched alkyl chain of 1-20 carbon atoms, unsubstituted or substituted with one or more hydroxyl, alkoxy, aryloxy, amino or substituted amino groups, fluoroalkane, p-fluoroaryl, deuterated alkyl groups, and mixtures thereof.
21. The micelle of aspect 20 which further includes an imaging agent and which micelle corresponds to the formula: 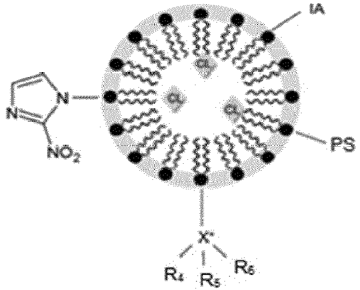 where IA is selected from the group consisting of fluorophores, dyes, quantum dots, transition metal, transition metal complexes, radionuclides, and mixtures thereof.
22. A method for treating a hypoxic tumor comprising;
   targeting the tumor with a polymeric micelle, the micelle corresponding to the micelle of aspect 20.
23. The method of aspect 22 wherein the, micelle further comprises an imaging agent moiety, the, micelle corresponding to the formula: 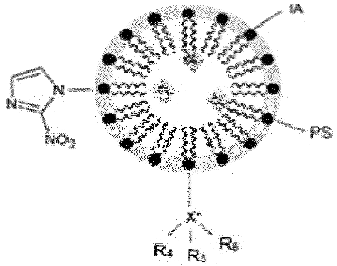
where IA is selected from the group consisting of fluorophores, dyes, quantum dots, transition metal, transition metal complexes, radionuclides, and mixtures thereof.

## Claims

1. A polymeric micelle corresponding to the formula: 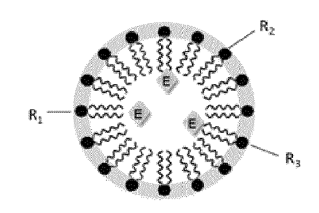
wherein E is an encapsulated agent within a polymeric micelle (PMC) in which E is selected from the group consisting of an imaging agent, a therapeutic agent, a therapeutic adjuvant, a light producing system, a radioactive system, a sensitizing agent, and mixtures thereof;
R₁ is a hypoxia targeting moiety including aromatic N-oxide, aliphatic N-oxide, nitroazole, nitroimidazole, nitrothiophene, nitrothiazole, nitrooxazole, nitrofuran, nitropyrrole, and transition metal moieties;
R₂ is selected from the group consisting of an imaging agent, a targeting moiety, a therapeutic agent, a sensitizing agent, and mixtures thereof;
R₃ is a polar biocompatible moiety for improving solubility, stability, and biodistribution of the micelle, and
wherein the sensitizing agent may be conjugated to the hypoxia targeting moiety.

2. The micelle of claim 1 wherein:
the polymeric micelle has a size ranging from about 10 nm to about 100 nm in diameter and comprises a hydrophobic biocompatible core and a hydrophilic or polar biocompatible corona, :
wherein the biocompatible polymer forming the core of the PMC is preferably selected from the group consisting of polystyrene, poly(divinylbenzene), poly(acrylate), polymethylmethacrylate, poly(hydroxyethyl methacrylate), poly(vinyltoluene), poly(butadiene), poly(aspartic acid), poly(benzyl aspartate), polycaprolactone and derivatives thereof, poly(lactide) and derivatives thereof, poly(benzyl glutamate), poly(L-lysine), poly(propylene oxide), oligo(methyl methacrylate), poly(isoprene), poly(isopropyl acrylamide), calixarenes, polyanhydrides, pseudo-poly(amino acids), polyphosphazenes and derivatives thereof and mixtures thereof.
and/or
the corona forming biocompatible polymer is preferably selected from the group consisting of, poly(ethylene glycol), poly(vinyl alcohol), poly(acrylic acid), poly(methacrylic acid), poly(acrylamide), poly(vinyl pyrrolidone), poly(ethylene oxide), poly(propylene oxide), poly(vinylmethyl ether), hydroxypropyl cellulose, chitosans, polysaccharides, tertiary ammonium and phosphonium salts, and mixtures thereof.

3. The micelle of any of the preceding claims which corresponds to the formula: 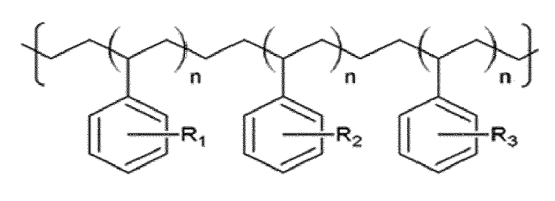 where n is an integer from 1 to 20.

4. The micelle of any of the preceding claims wherein:
the hypoxia targeting moiety is selected from the group consisting of aromatic N-oxide, aliphatic N-oxide, nitroazole, nitroimidazole, nitrothiophene, nitrothiazole, nitrooxazole, nitrofuran, nitropyrrole, transition metal moieties, and mixtures thereof.

5. The micelle of claim 5 wherein:
the imaging or contrast agent is selected from the group consisting offluorophores, dyes, quantum dots, transition metal, transition metal complexes, radionuclides, and mixtures thereof
and/or
the therapeutic agent is selected from the group consisting of chemotherapeutics, radioisotopes, therapeutic proteins or peptides, gene therapy, and mixtures thereof
and/or
the chemotherapeutic compound is selected from the group consisting of antimetabolites, alkylating agents, alkaloids, topoisomerase inhibitors, kinase inhibitors,
angiogenesis inhibitors, cytotoxic antibiotics, platinum based drugs and mixtures thereof
and/or
the chemotherapeutic compound is selected from the group consisting of antimetabolites, alkylating agents, alkaloids, topoisomerase inhibitors, kinase inhibitors,
angiogenesis inhibitors, cytotoxic antibiotics, platinum based drugs and mixtures thereof.
and/or
the sensitizing agent is selected from the group consisting of (a) photosensitizers, including naphthalene, anthracene, biphenyl, quinone, porphyrin, and phthalocyanins, (b) fluorescein, (c) Rose Bengal, (d) eosin blue, (e) erythrosin B, (f) oxygen carriers including endoperoxides and nitroxides, and (g) mixtures thereof.

6. The micelle of any of the preceding claims, wherein:
the polar biocompatible moiety is selected from the group consisting of poly(ethylene glycol), poly(vinyl alcohol), poly(acrylic acid), poly(methacrylic acid), poly(acrylamide), poly(vinyl pyrrolidone), poly(ethylene oxide), poly(propylene oxide), poly(vinylmethyl ether), hydroxypropyl cellulose, chitosans, polysaccharides, tertiary ammonium and phosphonium salts, and mixtures thereof.

7. The micelle of claim 6 wherein the polar biocompatible moiety is a tertiary salt corresponding to the formula: 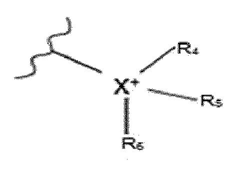 where X is either N or P; R₄, R₅, and R₆ are each,individually,a straight or branched alkyl chain of 1-20 carbon atoms, unsubstituted or substituted with one or more hydroxyl, alkoxy, aryloxy, amino or substituted amino groups, fluoroalkane, p-fluoroaryl, deuterated alkyl groups, and mixtures thereof.

8. The micelle of any of the preceding claims which corresponds to the formula: 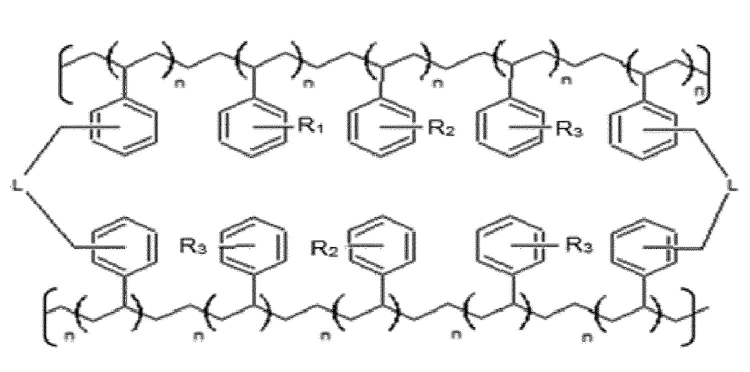 wherein L is a linker region of saturated or unsaturated carbons attached to trisubsituted amines or trisubstituted phosphines, and n is an integer from 1 to 20.

9. The micelle of claim 1 wherein R₁ corresponds to the formula: 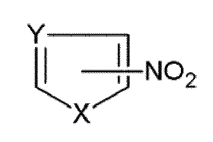 where X is N, S, or O and Y is C or N,
wherein the hypoxia targeting moiety is preferably a substituted or
unsubstituted 2-nitroimidazole and when both X and Y are Ncorresponds to the following: 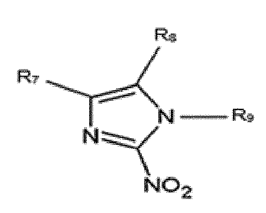 where R₇, R₈, and R₉, individually, represent attachment to a PMC directly or through a linker region; a deuterated or non-deuterated alkyl, a carboxylate, an alkyl carboxylate, an amino, a substituted or unsubstituted aryl, a substituted or unsubstitutedheteroaryl, and mixtures thereof.

10. The micelle of claim 6 which corresponds to formula: 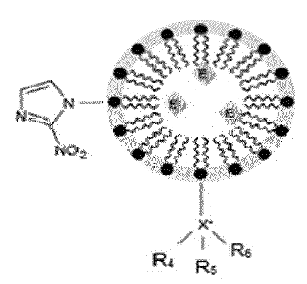 wherein 2-nitroimidazole is the targeting moiety, the polar biocompatible component is a tertiary salt, E is a therapeutic agent,X is either N or P, R₄, R₅, and R₆ are, individually, a straight or branched alkyl chain of 1-20 carbon atoms, unsubstituted or substituted with one or more hydroxyl, alkoxy, aryloxy, amino or substituted amino groups, fluoroalkane, p-fluoroaryl, deuterated alkyl groups, and mixtures thereof.

11. The micelle of claim 10 which further comprises an imaging agent, the moiety corresponding to the formula: 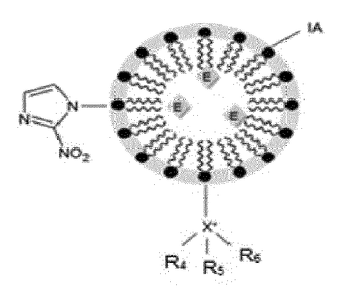 where IA represents an imaging agent including fluorescent moieties, deuterated moieties, electromagnetic moieties, radioisotopes, and mixtures thereof.

12. The micelle of claim 10 wherein the imaging agent is a fluorescent moietyselected from the group consisting of:
organic dyes, quantum dots, fluorescent probes, and fluorescent biomolecules, the imaging agents being covalently linked to the PMC either outside the PMC (hydrophilic), within the PMC (hydrophobic), or non-covalently linked to the PMC or encapsulated within the PMC (hydrophobic).

13. A photodynamic therapy polymeric micelle corresponding to the formula: 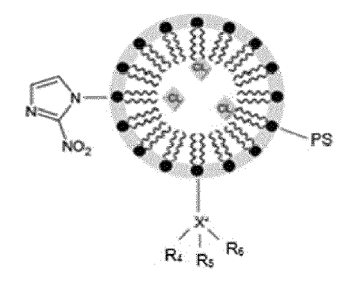 wherein the PMC is conjugated to 2-nitroimidazole, PS is a photosensitizer selected from the group consisting of napthalenes, anthracenes, biphenyls, quinones, porphyrins, phthalocyanins, fluorescein, fluorescein derivatives, Rose Bengal, eosin blue, and erythrosin B, X+ is a tertiary salt of either N or P, and CL is an encapsulated chemiluminescent substrate, the substrate being selected from the group consisting of substrates, 1,2-dioxetane compounds and luminol, 2-nitroimidazole, where the CL is triggered, R₄, R₅, and R₆ are, individually, a straight or branched alkyl chain of 1-20 carbon atoms, unsubstituted or substituted with one or more hydroxyl, alkoxy, aryloxy, amino or substituted amino groups, fluoroalkane, p-fluoroaryl, deuterated alkyl groups, and mixtures thereof.

14. The micelle of claim 13 which further includes an imaging agent and which micelle corresponds to the formula: 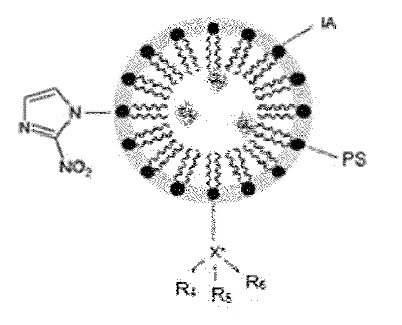 where IA is selected from the group consisting of fluorophores, dyes, quantum dots, transition metal, transition metal complexes, radionuclides, and mixtures thereof.

15. A method for treating a hypoxic tumor comprising;
targeting the tumor with a polymeric micelle, the micelle corresponding to the micelle of 13 or 14.
